# EUROPÄISCHE PATENTSCHRIFT

(11) **EP 2 560 746 B1**
(45) Veröffentlichungstag und Bekanntmachung des Hinweises auf die Patenterteilung: **14.11.2018**
(21) Anmeldenummer: 11718683.3
(22) Anmeldetag: 20.04.2011
(51) Int. Cl.: B01D 63/02, B01D 63/04, A61M 1/16

(54) **VERFAHREN UND VORRICHTUNG ZUR HERSTELLUNG EINES MEMBRANMODULS**
PROCESS AND APPARATUS FOR PRODUCING A MEMBRANE MODULE
PROCÉDÉ ET DISPOSITIF DE PRODUCTION D'UN MODULE MEMBRANAIRE

(30) Priorität: 20.04.2010 DE 102010027973
(43) Veröffentlichungstag der Anmeldung: 27.02.2013
(73) Patentinhaber: Xenios AG, 74076 Heilbronn (DE)
(72) Erfinder: STRAUSS, Andreas, 44801 Bochum (DE); GROSSER, Albert, 44801 Bochum (DE)
(74) Vertreter: Castell, Klaus
(86) Internationale Anmeldenummer: PCT/EP2011/056350
(87) Internationale Veröffentlichungsnummer: WO 2011/131729

(56) Entgegenhaltungen:
- EP-A2- 0 089 122
- WO-A2-00/44483
- US-A- 3 801 401
- US-A- 4 179 380
- US-A- 4 346 006

## Beschreibung

Die Erfindung betrifft ein Verfahren zur Herstellung eines Membranmoduls aus einer Matte aus Hohlfasern sowie eine Vorrichtung, die zur Durchführung dieses Verfahrens geeignet ist. Die Erfindung betrifft ferner ein Membranmodul aus Hohlfasern.

Im Rahmen der Behandlung von Patienten mit akutem Lungenversagen (ARDS), schweren respiratorischen Erkrankungen oder pulmonalen Insuffizienzen, aber auch in der kardiopulmonalen Chirurgie übernimmt in vielen Fällen ein externes Gerät, beispielsweise eine künstliche Lunge zeitweise die Funktionen der Lunge des Patienten. Ein wichtiger Bestandteil der künstlichen Lunge ist ein Oxygenator, in dem der erforderliche Gasaustausch zur Versorgung des Patienten mit Sauerstoff stattfindet.

Zu den verschiedenen Arten von Oxygenatoren gehören Filmoxygenatoren, Blasenoxygenatoren und Membranoxygenatoren, wobei in der modernen Medizin überwiegend nur noch Membranoxygenatoren im Verfahren der extrakorporalen Membranoxygenation (ECMO) zur Anwendung kommen.

Während einer ECMO-Behandlung wird dem Blut eines Patienten über einen Membranoxygenator lebenswichtiger Sauerstoff zugeführt und das durch Stoffwechselprozesse entstehende Kohlendioxid entfernt. Gas- und Blutseite sind dafür durch eine gasdurchlässige mikroporöse Membran voneinander getrennt und der Gasaustausch erfolgt durch Partialdruckdifferenzen entlang der Membran. Die Membranen sind häufig als semipermeable Hohlfasern ausgebildet, an denen vorzugsweise außen das Blut vorbeiströmt, während ein Sauerstoff-Luft-Gemisch die Hohlfasern von innen durchströmt. Gas- und Blutstrom können dabei beispielsweise im Gleich- oder Gegenstrom oder auch im Kreuzstrom zueinander geführt werden. An der Membran kommt es aufgrund eines Konzentrationsgefälles zum Austausch von Sauerstoff und Kohlendioxid. Das durchströmende Blut wird mit Sauerstoff angereichert und vom Kohlendioxid befreit. Die Trennung von Gas- und Blutseite ermöglicht eine Steuerung der Sauerstoffsättigung im Blut. Ein Membranoxygenator ist nur zum einmaligen Gebrauch bestimmt und wird nach der Anwendung entsorgt.

Das Kernstück eines Membranoxygenators bildet ein Hohlfaserbündel oder auch Membranmodul mit einer Trennschicht, das aus einer Vielzahl von einzelnen Hohlfasern gebildet wird, die an den jeweiligen Enden über eine Vergussmasse miteinander verbunden sind. Die EP 0 089 122 beispielsweise beschreibt ein Verfahren, bei dem aus gasdurchlässigen Hohlfasern ein Band hergestellt wird, das dann auf einen Wickelkern aufgewickelt und anschließend an seinen beiden Enden vergossen wird.

Für das Vergießen der Enden der Hohlfasern und das damit verbundene Ausbilden von vergossenen Randbereichen sind dabei insbesondere das Zentrifugalgießverfahren und das Tauchgießverfahren bekannt. Diese beiden Verfahren sind üblicherweise jedoch mit dem Nachteil verbunden, dass sie erst nach dem Aufwickeln der Hohlfasern auf den Wickelkern durchgeführt werden können. Es ist eine Übergabe der gewickelten Hohlfaserbündel an die Vorrichtung zum Vergießen der Enden erforderlich, bei der es zu Verschiebungen einzelner Lagen von Hohlfasern kommen kann.

Zu Verschiebungen einzelner Lagen von Hohlfasern kann es auch beim Einbringen der Trennschicht in das Hohlfaserbündel kommen, da diese indem gewickelten Hohlfaserbündel beispielsweise durch einen Hohlzylinder gebildet wird, der als separates Bauteil über einen Teil des gewickelten Hohlfaserbündels geschoben werden muss, bevor das Hohlfaserbündel bis auf seinen endgültigen Durchmesser gewickelt werden kann. Für das Aufschieben des Hohlzylinders muss der Wickelvorgang entsprechend unterbrochen werden.

Sowohl das separate Verfahren zum Vergießen der Enden der Hohlfasern als auch das Einbringen der Trennschicht in das Hohlfaserbündel erhöhen damit den Aufwand für die Fertigung des Membranmoduls, da der Wickelvorgang zum einen für das Aufschieben des Hohlzylinders auf das partiell gewickelte Hohlfaserbündel und zum anderen für die Übergabe des gewickelten Hohlfaserbündels an eine Vergießvorrichtung teilweise unterbrochen werden muss. Zudem kann dies zu Unterschieden in der Qualität der Membranmodule bis hin zu Fertigungsausfällen führen, da vollständig oder auch partiell gewickelte Hohlfaserbündel durch Verschiebungen einzelner Lagen von Hohlfasern während der nachfolgenden Verarbeitung möglicherweise nicht mehr brauchbar sind. Außerdem sind semipermeable Hohlfasermembranen mechanisch sehr empfindlich und können während der Verarbeitung beispielsweise beim Aufschieben des Hohlzylinders leicht beschädigt werden, was wiederum Ausfälle des Membranmoduls zur Folge haben kann.

Desweitern stehen mit den bekannten Verfahren zum Vergießen der Enden der Hohlfasern und zum Einbringen der Trennschicht in das Hohlfaserbündel nur begrenzte Möglichkeiten für die Ausgestaltung und Anordnung der Formen und Konturen für die vergossenen Randbereiche des Membranmoduls und für die Trennschicht im Membranmodul zur Verfügung. Die Variabilität für die Gestaltung der vergossenen Randbereiche und der Trennschicht ist entsprechend eingeschränkt.
Ein Verfahren und eine Vorrichtung zur Herstellung eines Membranmoduls mit den Merkmalen des Oberbegriffs des Patentanspruchs 1 bzw. des Oberbegriffs des Patentanspruchs 6 sind aus der US 3 801 401 A bekannt. Ähnliche Verfahren zeigen die DE WO 00/44483 A2 und die US 4 346 006. Ein Membranmodul mit den Merkmalen des Oberbegriffs des Patentanspruchs 7 ist aus der EP 0 089 122 A2 bekannt. US 4 179 380 beschreibt ein Hohlfasermodul.

Eine Aufgabe der Erfindung ist es daher, ein Verfahren bereitzustellen, mit dessen Hilfe Membranmodule mit einem geringen Aufwand für die Fertigung ohne große Schwankungen in der Qualität, ohne Fertigungsausfälle und mit einem strömungstechnisch günstigen Verlauf für den Blutstrom durch das Membranmodul und reduzierten Strömungswiderständen hergestellt werden können, bei denen einerseits Formen und Konturen sowie andererseits Anzahl und Anordnung sowohl für die vergossenen Randbereiche als auch für die Trennschicht variabel gestaltet werden können.

Eine Aufgabe der Erfindung ist es ferner, eine Vorrichtung zur Durchführung eines solchen Verfahrens sowie ein Membranmodul selbst bereitzustellen.

Diese Aufgabe wird gelöst durch ein Verfahren mit den Merkmalen des unabhängigen Anspruchs 1. Vorteilhafte Weiterbildungen des Verfahrens ergeben sich aus den Unteransprüchen 2 bis 5. Die Aufgabe wird ferner durch eine Vorrichtung nach Anspruch 6 gelöst Weiterhin wird die Aufgabe gelöst durch ein Membranmodul mit den Merkmalen des Anspruchs 7, für das sich eine vorteilhafte Ausführungsform aus dem Unteranspruch 8 ergibt.

Die Erfindung umfasst ein Verfahren zur Herstellung eines Membranmoduls aus einer Matte aus Hohlfasern, wobei mehrere Hohlfasern mit näherungsweise gleicher Länge nebeneinander angeordnet die Matte bilden. Die Matte wird in dem Verfahren bereichsweise mit einer Vergussmasse benetzt und auf einen zylinderförmigen Wickelkern aufgewickelt, wobei ein zylinderförmiger Körper mit einer Wandung aus Hohlfasern ausgebildet wird und wobei die Wandung aus Hohlfasern mindestens einen vergossenen Bereich aufweist.

In dem Verfahrens werden nach dem Benetzen von zwei sich gegenüberliegenden Randbereichen der Matte mit dem Aufwickeln der derart benetzten Matte zwei vergossene Randbereiche in der Wandung des zylinderförmigen Körpers ausgebildet.

Eine Ausgestaltung des Verfahrens sieht vor, dass eine Folie zur Ausbildung von mindestens einer Trennschicht in der Wandung des zylinderförmigen Körpers mit aufgewickelt wird. Bei einer anderen Ausgestaltung des Verfahrens wird partiell ein Mittelbereich der Matte mit Vergussmasse benetzt und mit dem Aufwickeln mindestens eine Trennschicht in der Wandung des zylinderförmigen Körpers ausgebildet.

In einer Ausgestaltung des Verfahrens wird partiell ein Endbereich der Matte benetzt und mit dem Aufwickeln eine Außenschicht in der Wandung des zylinderförmigen Körpers ausgebildet.

Jedem der zu benetzenden Bereiche der Matte wird in einer Ausführungsform des Verfahrens jeweils eine Benetzungskontur vorgegeben, die in Abhängigkeit von den Ergebnissen einer numerischen Strömungsberechnung definierbar ist.

Die Erfindung umfasst weiterhin eine Vorrichtung zur Herstellung eines Membranmoduls aus einer Matte aus Hohlfasern, wobei mehrere Hohlfasern mit näherungsweise gleicher Länge nebeneinander angeordnet eine Matte bilden. Die Vorrichtung weist neben einer Dosiereinrichtung für Vergussmasse, die die Matte bereichsweise benetzen kann, auch eine Spanneinrichtung zum Vorspannen der Matte sowie eine Wickeleinrichtung auf, die die vorgespannte Matte aufwickeln kann.

In einer Weiterbildung der Vorrichtung weist die Dosiereinrichtung eine Steuerungseinrichtung auf, die eine Benetzungskontur für einen zu benetzenden Bereich der Matte ermitteln und vorgeben kann und die weiterhin für den zu benetzenden Bereich mit der ermittelten Benetzungskontur eine Dosiermenge für die Vergussmasse bestimmen und vorgeben kann.

Die Erfindung umfasst ferner ein Membranmodul aus Hohlfasern, bei dem mehrere Hohlfasern mit näherungsweise gleicher Länge nebeneinander angeordnet eine Matte bilden, die wiederum bereichsweise mit einer Vergussmasse benetzt und auf einen zylinderförmigen Wickelkern aufgewickelt wurde. Die Matte bildet dann bereichsweise eine Wandung des Membranmoduls mit mindestens einem vergossenen Bereich.

In einer Weiterbildung des Membranmoduls weist die Wandung zwei vergossene Randbereiche sowie mindestens eine Trennschicht auf.

Bei einer Ausführungsform des Membranmoduls wird die Trennschicht durch eine Folie gebildet. Bei einer anderen Ausführungsform des Membranmoduls hingegen wird die Trennschicht durch eine Vergussmasse gebildet.

In einer Weiterbildung des Membranmoduls umfasst die Wandung eine Außenschicht, die durch eine Vergussmasse gebildet wird.

In einer Ausgestaltung des Membranmoduls weisen die Konturen der vergossenen Bereiche, die in einem Längsschnitt durch die Wandung des Membranmoduls sichtbar sind, bereichsweise eine Krümmung auf.

Hierbei wurde das Membranmodul nach dem Verfahren der Erfindung hergestellt.

Das beschriebene Verfahren und die Vorrichtung zur Herstellung eines Membranmoduls sowie das Membranmodul selbst haben den Vorteil, dass die Ausbildung der vergossenen Randbereiche und der Trennschicht ohne Unterbrechungen des Fertigungsflusses möglich ist. Sowohl die vergossenen Randbereiche als auch die Trennschicht werden unmittelbar während des Wickelvorganges mit ausgebildet, da die Vergussmasse direkt auf die Matte, aus der das Membranmodul durch entsprechendes Aufwickeln gebildet wird, aufgebracht wird. Dadurch wird das Verfahren einfach und kostengünstig.

Die Erfindung wendet sich somit ab von Verfahren, bei denen die Enden der Hohlfasern erst nach dem Wickelvorgang vergossen werden. Sie wendet sich zudem ab von Verfahren, bei denen die Trennschicht im Membranmodul durch einen Hohlzylinder ausgebildet wird, der nur während einer Unterbrechung des Wickelvorganges in das Hohlfaserbündel eingebracht werden kann. Der Fertigungsprozess ist somit einfacher und zudem sicherer, da Qualitätsschwankungen durch verschobene Lagen von Hohlfasern vermieden werden.

Ein weiterer Vorteil der Erfindung zeigt sich darin, dass beliebige Formen und Konturen sowohl für die vergossenen Randbereiche als auch für die Trennschicht des Membranmoduls ausgebildet werden können. Zudem ist dabei sowohl die Anzahl als auch die Anordnung der auszubildenden vergossenen Bereiche innerhalb des Membranmoduls frei definierbar. Für die Randbereiche und die Trennschicht können somit optimierte Strömungskonturen für reduzierte Strömungswiderstände umgesetzt werden, die einen schonenden Durchfluss des Blutes bewirken.

Die Erfindung wendet sich somit ebenfalls ab von Verfahren, bei denen eine Variabilität in der Gestaltung, Anordnung und Anzahl der Randbereiche und Trennschicht(en) nicht gewährleistet werden kann.

Die zuvor genannten und weitere Vorteile, Besonderheiten und zweckmäßige Weiterbildungen der Erfindung werden auch anhand der Ausführungsbeispiele deutlich, die nachfolgend unter Bezugnahme auf die Figuren beschrieben werden.

Von den Figuren zeigt:
Fig. 1a eine Matte aus Hohlfasern,
Fig. 1b eine doppellagige Matte aus Hohlfasern,
Fig. 2a eine doppellagige Matte aus Hohlfasern mit zwei benetzten Randbereichen,
Fig. 2b eine doppellagige Matte aus Hohlfasern mit zwei benetzten Randbereichen und einem benetzten Mittelbereich,
Fig. 2c eine doppellagige Matte aus Hohlfasern mit zwei benetzten Randbereichen und zwei benetzten Mittelbereichen,
Fig. 3 ein Membranmodul,
Fig. 4a einen Querschnitt durch ein Membranmodul mit einer Trennschicht,
Fig. 4b einen Längsschnitt durch ein Membranmodul mit einer Trennschicht,
Fig. 4c einen Längsschnitt durch ein Membranmodul mit einer Trennschicht und einer Außenschicht,
Fig. 5a einen Querschnitt durch ein Membranmodul mit einer Trennschicht mit mehreren Bereichen und
Fig. 5b einen Längsschnitt durch ein Membranmodul mit einer Trennschicht mit mehreren Bereichen.

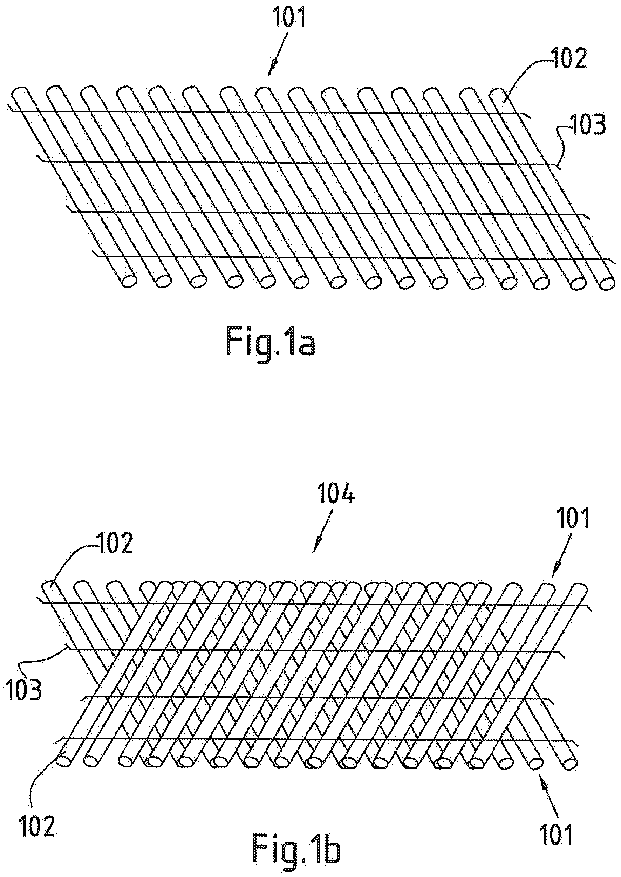

In Fig. 1a ist schematisch eine Matte 101 aus Hohlfasern 102, die jeweils zwei verschlossene Enden sowie ein Mittelstück aufweisen, dargestellt. Die Hohlfasern 102 sind näherungsweise parallel nebeneinander mit näherungsweise gleich großen Abständen zueinander angeordnet und mittels Querfäden 103 miteinander verbunden. Querfäden 103 und Hohlfasern 102 stehen dabei in einem Winkel zueinander, der zwischen 0° und 90° liegen kann. Die Querfäden 103 halten die Hohlfasern 102 in einer vorgegebenen Position und erleichtern so die weitere Verarbeitung der Matte 101, wobei die Herstellung einer derartigen Matte 101 mit einem der im Stand der Technik bekannten Verfahren beispielsweise auf einer Web- oder Wirkmaschine erfolgen kann.

Zwei der Matten 101 übereinander gelegt bilden eine doppellagige Matte 104 wie in Fig. 1b schematisch als Beispiel dargestellt. Die beiden Matten 101 sind hier so angeordnet, dass sich durch die übereinander liegenden Hohlfasern 102 der Matten 101 eine Gitterstruktur ausbildet.

Mit einer ebenfalls möglichen Anordnung von mehr als zwei Matten 101 übereinander kann auch eine mehrlagige Matte für die weitere Verarbeitung ausgebildet werden. Bei deren Herstellung ist dann darauf zu achten, dass sich die Gitterstruktur entsprechend zwischen jeweils unmittelbar aufeinanderliegenden Matten 101 wiederholt.

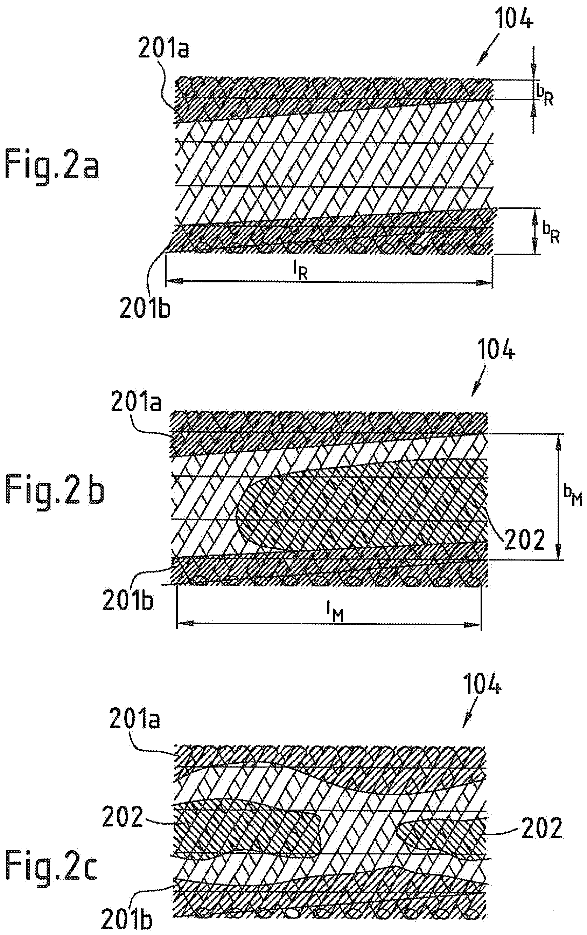

In Fig. 2a ist schematisch das Beispiel einer doppellagigen Matte 104 mit zwei gegenüberliegenden Randbereichen 201a und 201b dargestellt, die von den aufeinanderliegenden Endender Hohlfasern 102 der Matten 101 gebildet werden und jeweils mit einer Vergussmasse benetzt sind.

Die benetzten Randbereiche 201a und 201b werden jeweils über die veränderbaren Parameter Breite bR sowie Länge lR definiert. Dabei ist es möglich, dass die jeweilige Breite bR über deren zugehörige Länge lR ungleichförmig mit wechselnden quantitativen Werten verläuft, sodass sich unterschiedliche Benetzungskonturen für die beiden benetzten Randbereiche 201a und 201b ergeben können, die sich als unregelmäßige, benetzte Streifen mit variabler Breite auf der doppellagigen Matte 104 abbilden. Verläuft die Breite bR über deren zugehörige Länge 1R hingegen gleichförmig mit einem konstanten quantitativen Wert, erscheint die Benetzungskontur als Streifen mit gleichbleibender Breite auf der doppellagigen Matte 104.

In dem in Fig. 2b schematisch dargestellten Beispiel einer doppellagigen Matte 104 ist zusätzlich zu den beiden Randbereichen 201a und 201b auch ein Mittelbereich 202 der doppellagigen Matte 104, der aus den aufeinanderliegenden Mittelstücken der Hohlfasern 102 der Matten 101 gebildet wird, bereichsweise mit der Vergussmasse benetzt. Der benetzte Randbereich 201a sowie der benetzte Mittelbereich 202 gehen hier teilweise beispielhaft ineinander über, während zwischen dem benetzten Randbereich 201b und dem benetzten Mittelbereich 202 beispielhaft ein nicht benetzter Bereich der doppellagigen Matte 104 sichtbar ist.

Der benetzte Mittelbereich 202 wird über die veränderbaren Parameter Breite bM und Länge lM definiert. Auch hier besteht die Möglichkeit, dass die Breite bM des benetzten Mittelbereiches 202 ungleichförmig mit wechselnden quantitativen Werten über die Länge lM verläuft und dass sich dadurch eine entsprechend festgelegte Benetzungskontur für den Mittelbereich 202 ergibt. Bei einem gleichförmigen Verlauf der Breite bM des benetzten Mittelbereiches 202 über die Länge lM mit konstanten quantitativen Werten erscheint die Benetzungskontur hingegen als Streifen mit einer gleichbleibenden Breite auf der doppellagigen Matte 104 .

Bedingt durch die veränderbaren Parameter für die benetzten Bereiche ist es möglich, unterschiedliche Benetzungskonturen abzubilden, deren Formen variabel und frei wählbar sind. Für die Abbildung der Benetzungskonturen auf den zu benetzenden Bereichen durch die Vergussmasse können beispielsweise auf die doppellagige Matte 104 aufgelegte Schablonen verwendet werden, sodass nur die durch die Schablone sichtbaren Bereiche der doppellagigen Matte 104 benetzbar sind. Es ist jedoch auch möglich, für die Vergussmasse eine Dosiereinrichtung mit einer Steuerungseinrichtung einzusetzen. Mittels dieser Steuerungseinrichtung können der Dosiereinrichtung die Benetzungskonturen direkt vorgegeben werden, die diese entsprechend auf der doppellagigen Matte 104 während der Benetzung nachbildet.

Fig. 2c zeigt schematisch das Beispiel einer doppellagigen Matte 104 mit jeweils zwei gegenüberliegenden Randbereichen 201a und 201b und Mittelbereichen 202, die jeweils mit Vergussmasse benetzt sind. Jede Benetzungskontur weist eine eigene Form auf, die sich beispielhaft von den Benetzungskonturen der anderen benetzten Bereiche unterscheidet. Es können aber auch gleiche Benetzungskonturen für unterschiedliche benetzte Bereiche auftreten.

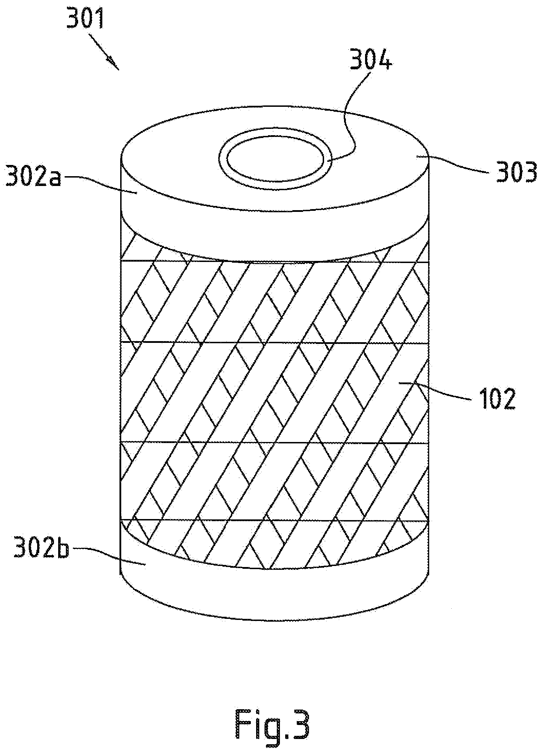

In der Weiterverarbeitung wird die bereichsweise benetzte doppellagige Matte 104 mittels einer Spanneinrichtung vorgespannt, dann mittels einer Wickelmaschine aufgewickelt und so ein Membranmodul 301 ausgebildet, das schematisch in Fig. 3 dargestellt ist. Das Beispiel des Membranmoduls 301 stellt sich als ein zylinderförmiger Körper dar, der eine Wandung 303 aus Hohlfasern 102 mit zwei vergossenen Randbereichen 302a und 302b aufweist. Gebildet werden die vergossenen Randbereiche 302a und 302b durch das Übereinanderlagern der benetzten Randbereiche 201a und 201b beim Aufwickeln der doppellagigen Matte 104 auf einen Wickelkern 304, der sich im Inneren des zylinderförmigen Körpers befindet. Der Wickelkern 304 kann dabei beispielsweise als Zylinder oder auch als Hohlzylinder ausgebildet sein. Zudem ist es möglich, den Wickelkern 304 einteilig oder aus mehreren Teilen auszubilden. Bei mehreren Teilen ist sowohl eine radiale als auch eine axiale Teilung des Wickelkerns 304 möglich.

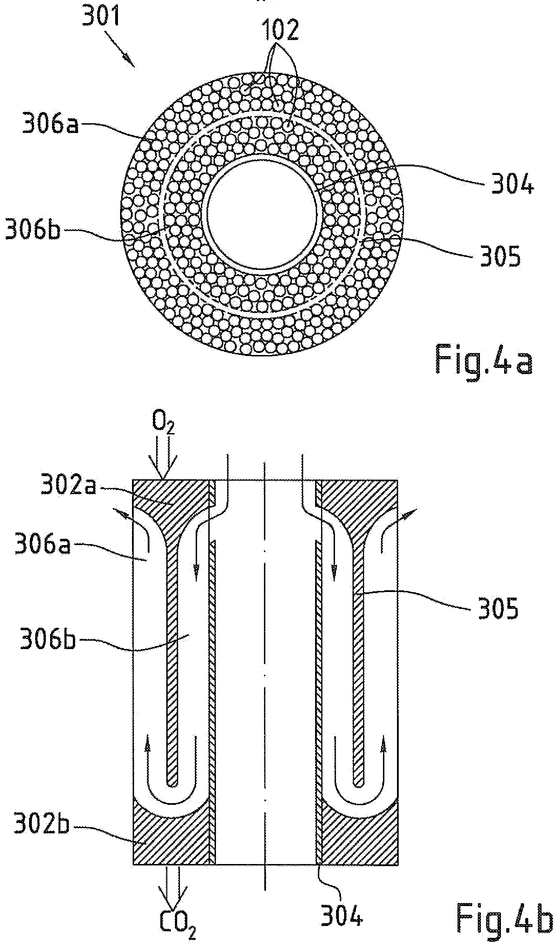

In Fig. 4a ist schematisch ein Querschnitt durch ein Membranmodul 301 mit einer Trennschicht 305 dargestellt, der den schematischen Aufbau der Wandung 303 des Membranmoduls 301 mit den Wandungsbereichen 306a und 306b in dem Membranmodul 301 zeigt. Zwischen den beiden Wandungsbereichen 306a und 306b, die jeweils aus aneinander liegenden Hohlfasern 102 gebildet werden, liegt die Trennschicht 305, die die beiden Wandungsbereiche 306a und 306b bereichsweise voneinander trennt. Im Innenbereich des Membranmoduls 301 befindet sich der Wickelkern 304, auf den die doppellagige Matte 104 aufgewickelt wurde.

Fig. 4b zeigt schematisch einen Längsschnitt durch ein Membranmodul 301 mit einer Trennschicht 305, in dem die Konturen der vergossenen Randbereiche 302a und 302b sowie der Trennschicht 305 dargestellt sind. Pfeile verdeutlichen die Strömungsrichtung des Blutes durch die Wandungsbereiche 306a und 306b des Membranmoduls 301 . Das Blut strömt dabei durch die von den Hohlfasern 102 gebildete Gitterstruktur an der Außenseite der Hohlfasern 102 entlang.

Ebenfalls entsprechend durch Pfeile gekennzeichnet ist der Gasstrom durch das Membranmodul 301, bei dem ein Sauerstoff-Luft-Gemisch mit einem höheren Anteil Sauerstoff (O2) zugeführt und mit einem höheren Anteil Kohlendioxid (CO2) abgeführt wird. Der Gasstrom wird dabei durch das Innere der Hohlfasern 102 des Membranmoduls 301 geführt. An der Membran der Hohlfasern 102 kommt es aufgrund der Konzentrationsunterschiede von Sauerstoff und Kohlendioxid im Blut und im Sauerstoff-Luft-Gemisch zum Austausch von Sauerstoff und Kohlendioxid. Das durchströmende Blut wird mit Sauerstoff angereichert und gleichzeitig vom Kohlendioxid befreit.

Die vergossenen Randbereiche 302a und 302b lassen jeweils eine Kontur erkennen, die bereichsweise eine oder mehrere Krümmungen aufweist. Diese Krümmungen haben einen wesentlichen Einfluss auf das Strömungsverhalten des Blutes durch das Membranmodul 301 . Trifft ein Blutstrom auf eine gerade Fläche oder eine eckige Kante, bilden sich üblicherweise sogenannte Totwassergebiete, die zu einer Rezirkulation des Blutes und zu einer Thrombenanlagerung an umliegenden Wänden führen, da das Blut nicht vollständig weitergeleitet wird. Die Krümmungen der vergossenen Randbereiche 302a und 302b verhindern jedoch teilweise oder sogar vollständig die Entstehung von Totwassergebieten, sodass sich mögliche Blutschädigungen sowie auftretende Thromboseneigungen verringern. Zudem wird verhindert, dass der Aufprall der festen Bestandteile des Blutes auf einer geraden Fläche eine Blutschädigung bewirkt.

Ein strömungstechnisch günstiger Verlauf für den Blutstrom durch das Membranmodul 301 wird mit Verfahren der numerischen Strömungsmechanik (engl.: CFD - Computational Fluid Dynamics) berechnet. Die Ergebnisse eines derart berechneten Verlaufes werden dafür verwendet, optimierte Strömungskonturen für die vergossenen Randbereiche 302a und 302b sowie für die Trennschicht 305 festzulegen.

Die Konturen der vergossenen Randbereiche 302a und 302b ergeben sich automatisch aus den Benetzungskonturen der Randbereiche 201a und 201b durch das Übereinanderlagern der Hohlfasern 102 beim Aufwickeln der doppellagigen Matte 104. Zur Nachbildung der festgelegten optimierten Strömungskonturen für die vergossenen Randbereiche 302a und 302b ist es erforderlich, insbesondere die veränderbaren Parameter Breite bR und Länge lR für die Benetzungskonturen der Randbereiche 201a und 201b der doppellagigen Matte 104 aus den optimierten Strömungskonturen abzuleiten. Hierfür stehen unterschiedliche Computerprogramme zur Verfügung, mit denen die veränderbaren Parameter ermittelt werden können.

Die eigentliche Ausbildung der vergossenen Randbereiche 302a und 302b mit den optimierten Strömungskonturen erfolgt somit durch das Benetzen der Randbereiche 201a und 201b der doppellagigen Matte 104 mit Vergussmasse entsprechend der ermittelten Benetzungskontur und durch das zugehörige Aufwickelnder benetzten doppellagige Matte 104.

Aus der Benetzungskontur des Mittelbereichs 202 ergibt sich automatisch durch das Übereinanderlagern der Hohlfasern 102 beim Aufwickeln der doppellagigen Matte 104 die Kontur der sich dadurch ausbildenden Trennschicht 305. Hier sind die aus der optimierten Strömungskontur zu bestimmenden Parameter für die Benetzungskontur insbesondere die variierende Breite bM über die zu berücksichtigende Länge lM des benetzten Mittelbereiches 202. Insbesondere die Länge lM wird dabei bestimmt durch die gewünschte Stärke der Trennschicht 305.

Ebenfalls für die Trennschicht 305 zu ermitteln sind die Parameter für die Position, an der die Trennschicht 305 in dem Membranmodul 301 ausgebildet werden soll. Diese Position wiederum bildet die Grundlage für die Festlegung der Position der Benetzungskontur des benetzten Mittelbereichs 202 auf der doppellagigen Matte 104. Zur Ausbildung der Trennschicht 305 wird die doppellagige Matte 104 dann entsprechend der ermittelten Benetzungskontur des Mittelbereiches 202 an der ermittelten Position mit der Vergussmasse benetzt und aufgewickelt.

Ein weiteres Beispiel für die Ausbildung der Trennschicht 305 ist die Verwendung einer Folie anstelle der Vergussmasse. Diese Folie wird beispielsweise durch Zuschneiden oder Stanzen in die ermittelte Kontur für die Ausbildung der Trennschicht 305 gebracht, entsprechend den ermittelten Parametern auf die doppellagige Matte 104 aufgebracht und positioniert und anschließend mit der doppellagigen Matte 104 aufgewickelt.

Geeignete Folienmaterialien sind beispielsweise Polyethylen (PE) oder Polypropylen (PP). Daneben eignen sich aber auch Polyvinylchlorid (PVC), Polystyrol (PS), verschiedene Polyester sowie Polycarbonat (PC) als Folienmaterial.

Die erforderliche Stärke für die Trennschicht 305 lässt sich unter anderem aus der geplanten Anwendung des Membranmoduls 301 in einem Membranoxygenator ableiten. Da die frei verfügbare Membranoberfläche zum Austausch von Sauerstoff und Kohlendioxid bei Membranoxygenatoren mit einer stark ausgebildeten Trennschicht 305 geringer ist, sind derartige Membranoxygenatoren besser geeignet für Kinder. Membranoxygenatoren mit einer nicht so stark ausgebildeten Trennschicht 305 hingegen sind aufgrund ihrer größeren frei verfügbaren Membranoberfläche besser geeignet für Erwachsene.

Die frei verfügbare Membranoberfläche lässt sich dabei allerdings nicht nur über die Stärke der Trennschicht 305, sondern auch über die Breite der beiden Randbereiche 302a, b beeinflussen. Und auch eine entsprechende Kombination von Stärke der Trennschicht 305 und Breite der beiden Randbereiche 302a, b zur Festlegung der frei verfügbaren Membranoberfläche ist möglich.

Für unterschiedliche Anwendungsgebiete ist es somit nicht zwingend erforderlich, Hohlfaserbündel mit verschiedenen Durchmessern herzustellen, da die Stärke der Trennschicht 305 und/oder die Breite der Randbereiche 302a, b während der Fertigung des Hohlfaserbündels frei wähl- und einstellbar ist. Das schließt jedoch nicht aus, dass auch Hohlfaserbündel mit verschiedenen Durchmessern gefertigt werden können.

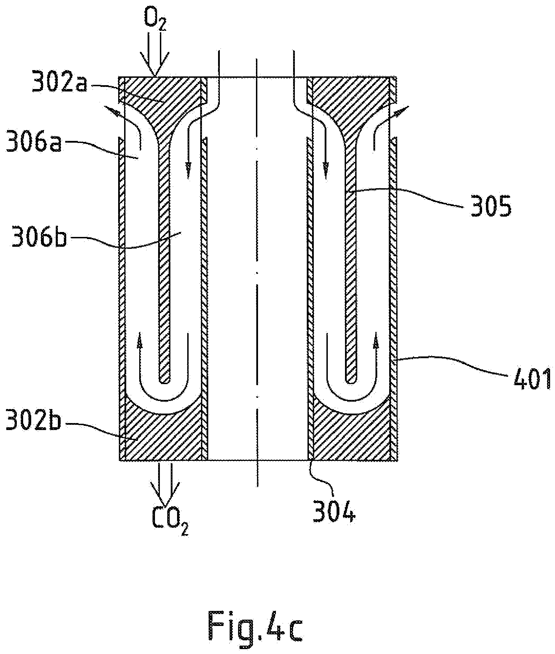

In Fig. 4c ist schematisch das Beispiel eines Längsschnitts durch ein Membranmodul 301 mit einer Trennschicht 305 sowie mit einer Außenschicht 401 dargestellt, deren Ausbildung in gleicher Weise erfolgt, wie die Ausbildung der Trennschicht 305 und der vergossenen Randbereiche 302a und 302b durch eine entsprechende Benetzung ermittelter und definierter Bereiche der doppellagigen Matte 104 mit der Vergussmasse.

Die derart ausgebildete Außenschicht 401 führt zu einer äußeren Abdichtung des Membranmoduls 301, ein zusätzliches Gehäuseteil ist somit nicht erforderlich. Damit entfällt auch der zusätzliche Montageschritt für das Einbringen des Membranmoduls 301 in ein Gehäuseteil. Dies wiederum führt zu einer Schonung der mechanisch empfindlichen Hohlfasern. Außerdem kann durch die Ausbildung der Außenschicht 401 eine Randgängigkeit des Blutes im Außenbereich des Membranmoduls 301 vermieden und damit eine Schonung des Blutes während des Durchflusses durch das Membranmodul 301 bewirkt werden.

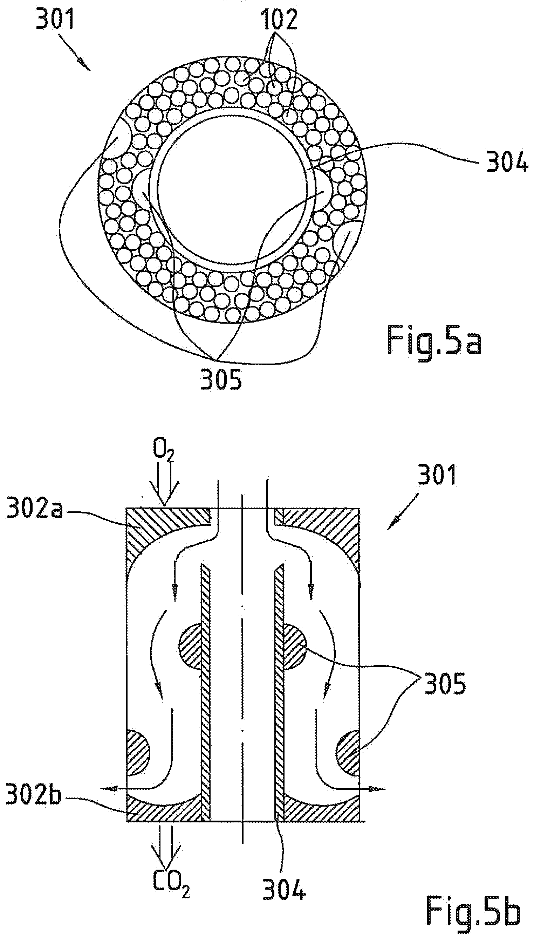

Mit der Integration einer Trennschicht 305 mit mehreren Bereichen in das Membranmodul 301 kann ein niedriger Druckverlust im Membranmodul 301 erreicht und damit der Durchfluss des Blutes noch schonender gestaltet werden. In Fig. 5a ist schematisch ein Querschnitt durch ein Membranmodul 301 mit einer Trennschicht 305 mit mehreren Bereichen dargestellt, die in Abhängigkeit von den berechneten günstigen Strömungsverläufen beliebig in dem Membranmodul 301 angeordnet sind.

Fig. 5b zeigt schematisch einen Längsschnitt durch ein Membranmodul 301 mit einer Trennschicht 305 mit mehreren Bereichen, der die Konturen und Positionen der vergossenen Randbereiche 302a und 302b sowie der Bereiche der Trennschicht 305 sichtbar macht. Pfeile verdeutlichen auch hier die Strömungsrichtung des Blutes durch das Membranmodul 301. Ebenfalls entsprechend durch Pfeile gekennzeichnet ist der Gasstrom durch das Membranmodul 301.

Durch die im Membranmodul 301 ausgebildeten Bereiche der Trennschicht 305 wird die Entstehung möglicher Totwassergebiete weiter reduziert und das Blut wird sehr schonend durch das Membranmodul 301 geleitet. Da dabei eine Schädigung der Blutbestandteile weitgehend verhindert wird, verringert sich so auch das Risiko von auftretenden Thrombosen.

Die wichtigsten Verfahrensschritte bei der Herstellung eines Membranmoduls 301 werden bestimmt durch das Benetzen und das Aufwickeln der doppellagigen Matte 104. Dabei besteht einerseits die Möglichkeit, die doppellagige Matte 104 erst zu benetzen und anschließend aufzuwickeln. Es ist andererseits aber auch möglich, in Abhängigkeit von den verwendeten Dosier- und Wickeleinrichtungen das Benetzen während des Aufwickelns vorzunehmen.

Als Vergussmasse zum Benetzen der doppellagigen Matte 104 sind verschiedene Kunstharze vorstellbar, deren Aushärtung durch eine chemische Reaktion erfolgt, die beispielsweise durch Wärme, zugesetzte Härter, Ultraviolettstrahlung oder auch Feuchtigkeit ausgelöst werden kann. Beispiele für verwendbare Kunstharze sind Polyesterharz, Polyurethanharz, Epoxidharz, Silikonharz oder Vinylesterharz.

Weitere Möglichkeiten für eine verwendbare Vergussmasse sind physikalisch abbindende oder auch chemisch härtende Klebstoffe sowie künstliche oder natürliche Wachse. Besonders zu berücksichtigende Bedingungen für die Auswahl einer geeigneten Vergussmasse sind dabei beispielsweise eine Biokompatibilität oder auch die Temperaturbeständigkeit.

Das Verfahren zur Herstellung des Membranmoduls 301 kann sich einerseits auf die Verwendung einer Vergussmasse für alle zu benetzenden Bereiche beschränken. Es besteht jedoch auch die Möglichkeit, mehrere verschiedene Vergussmassen zu verwenden oder Folie und Vergussmasse für die Ausbildung der einzelnen Schichten und Bereiche zu kombinieren.

Bevor das Membranmodul 301 mit den vergossenen Bereichen einer weiteren Verwendung zugeführt wird, erfolgt noch ein Abschneiden der verschlossenen Enden der Hohlfasern 102 des Hohlfaserbündels. Dadurch wird sichergestellt, dass einerseits der Gasstrom ungehindert durch die Hohlfasern 102 hindurch strömen kann und dass andererseits die Abmessungen des Membranmoduls 301 den Vorgaben entsprechen.

Es ist zu beachten, dass das Herstellungsverfahren des Membranmoduls 301 nicht auf die Verwendung einer bereichsweise benetzten doppellagigen Matte 104 begrenzt ist. In gleicher Weise dafür verwendbar sind sowohl eine einlagige Matte 101 als auch eine mehrlagige Matte. Ebenfalls möglich erscheint die Verwendung einer Hohlfaser 102, die vor der Verarbeitung erst zu einer Matte 101 verarbeitet wird, bevor die Weiterverarbeitung zu einem Membranmodul 301 erfolgt.

Weiterhin ist anzumerken, dass die Verwendung eines Membranmoduls 301, das nach dem beschriebenen Verfahren hergestellt wurde, nicht nur auf einen Einsatz in einem Membranoxygenator einer künstlichen Lunge beschränkt ist. Weitere mögliche Anwendungsgebiete für das Membranmodul 301 ergeben sich beispielsweise in der Dialyse oder auch in der Biotechnologie oder Pharmazie.

### Bezugszeichenliste

101 Matte, 102 Hohlfaser, 103 Querfaden, 104 doppellagige Matte, 201a, b benetzte Randbereiche, 202 benetzter Mittelbereich, 301 Membranmodul, 302a, b vergossene Randbereiche, 303 Wandung, 304 Wickelkern, 305 Trennschicht, 306a, b Wandungsbereiche, 401 Außenschicht bRBreite der benetzten Randbereiche, 201a, b lRLänge der benetzten Randbereiche, 201a, b bMBreite des benetzten Mittelbereiches, 202 lMLänge des benetzten Mittelbereiches 202

## Patentansprüche

1. Verfahren zur Herstellung eines Membranmoduls (301) aus einer Matte (101) aus Hohlfasern (102) für einen strömungstechnisch günstigen Blutstromverlauf durch das Membranmodul, wobei mehrere Hohlfasern (102) mit näherungsweise gleicher Länge nebeneinander angeordnet die Matte (101) bilden, wobei die Matte (101) bereichsweise mit einer Vergussmasse benetzt wird und dass die bereichsweise mit der Vergussmasse benetzte Matte (101) auf einen zylinderförmigen Wickelkern (304) aufgewickelt wird, wobei ein zylinderförmiger Körper mit einer Wandung (303) aus Hohlfasern (102) ausgebildet wird und wobei die Wandung (303) aus Hohlfasern (102) mindestens einen vergossenen Bereich aufweist, wobei für jeden der zu benetzenden Bereiche der Matte (101) jeweils eine Benetzungskontur vorgegeben wird, ***dadurch gekennzeichnet, dass*** die Benetzungskontur in Abhängigkeit von den Ergebnissen einer numerischen Strömungsberechnung definiert wird, die **einen** strömungstechnisch günstigen Blutstromverlauf berechnet.

2. Verfahren nach Anspruch 1, bei dem zwei sich gegenüberliegende Randbereiche (201a , b) der Matte (101) mit der Vergussmasse benetzt werden und mit dem Aufwickeln zwei vergossene Randbereiche (302a, b) in der Wandung (303) des zylinderförmigen Körpers ausgebildet werden.

3. Verfahren nach Anspruch 2, bei dem partiell ein Mittelbereich (202) der Matte (101) benetzt und mit dem Aufwickeln mindestens eine Trennschicht (305) in der Wandung (303) des zylinderförmigen Körpers ausgebildet wird.

4. Verfahren nach einem der Ansprüche 1 bis 3, bei dem partiell ein Endbereich der Matte (101) benetzt und mit dem Aufwickeln eine Außenschicht (401) in der Wandung (303) des zylinderförmigen Körpers ausgebildet wird.

5. Verfahren nach Anspruch 2, bei dem eine Folie zur Ausbildung von mindestens einer Trennschicht (305) in der Wandung (303) des zylinderförmigen Körpers mit aufgewickelt wird.

6. Vorrichtung zur Herstellung eines Membranmoduls (301) aus einer Matte (101) aus Hohlfasern (102) für einen strömungstechnisch günstigen Blutstromverlauf durch das Membranmodul, wobei mehrere Hohlfasern (102) mit näherungsweise gleicher Länge nebeneinander angeordnet die Matte (101) bilden, wobei die Vorrichtung eine Dosiereinrichtung für Vergussmasse aufweist, wobei diese Dosiereinrichtung so ausgebildet ist, dass sie die Matte (101) bereichsweise benetzen kann, dass die Vorrichtung eine Spanneinrichtung aufweist, wobei diese Spanneinrichtung so ausgebildet ist, dass sie die Matte (101) vorspannen kann und dass die Vorrichtung eine Wickeleinrichtung aufweist, wobei diese Wickeleinrichtung so ausgebildet ist, dass sie die vorgespannte Matte (101) aufwickeln kann, ***dadurch gekennzeichnet, dass*** die Dosiereinrichtung eine Steuerungseinrichtung aufweist, die so ausgebildet ist, dass sie eine Kontur für einen zu benetzenden Bereich der Matte (101) ermitteln und vorgeben kann und dass sie für den zu benetzenden Bereich mit der ermittelten Kontur eine Dosiermenge für die Vergussmasse bestimmen und vorgeben kann, wobei für jeden der zu benetzenden Bereiche der Matte (101) jeweils eine Benetzungskontur vorgegeben wird, die in Abhängigkeit von den Ergebnissen einer numerischen Strömungsberechnung definiert wird, die **einen** strömungstechnisch günstigen Blutstromverlauf berechnet.

7. Membranmodul (301) aus Hohlfasern (102), wobei mehrere Hohlfasern (102) mit näherungsweise gleicher Länge nebeneinander angeordnet eine Matte (101) bilden, wobei die Matte (101), nachdem sie bereichsweise mit einer Vergussmasse benetzt und auf einen zylinderförmigen Wickelkern (304) aufgewickelt wurde, bereichsweise eine Wandung (303) des Membranmoduls (301) mit mindestens einem vergossenen Bereich bildet, ***dadurch gekennzeichnet, dass*** die Konturen der vergossenen Bereiche, die in einem Längsschnitt durch die Wandung (303) sichtbar sind, bereichsweise eine Krümmung aufweisen, wobei die Wandung (303) zwei vergossene Randbereiche (302a,b) sowie mindestens eine Trennschicht (305) aufweist, wobei ferner die Trennschicht (305) durch eine Vergussmasse gebildet wird, wobei das Membranmodul nach einem Verfahren nach Anspruch 1 hergestellt wurde.

8. Membranmodul (301) nach Anspruch 7, bei dem die Wandung (303) eine Außenschicht (401) aufweist, die durch eine Vergussmasse gebildet wird.

## Claims

1. A method for producing a membrane module (301) from a mat (101) made of hollow fibers (102) for a favorable fluidic blood current flow through the membrane module, wherein a plurality of hollow fibers (102) which have approximately the same length are arranged next to one another to form the mat (101), wherein the mat (101) is wetted in regions with a casting compound, and the mat (101), wetted in regions with the casting compound, is wound on a cylindrical winding core (304), wherein a cylindrical body is formed with a wall (303) made of hollow fibers (102), and wherein the wall (303) made of hollow fibers (102) has at least one molded region, wherein for each of the regions of the mat (101) to be wetted, a wetting contour is respectively defined, ***characterized in that*** the wetting contour is defined as a function of the results of a numeric fluid flow analysis which calculates a favorable fluidic blood current flow.

2. The method according to Claim 1, in which two opposite edge areas (201a, b) of the mat (101) are wetted with the casting compound and two molded edge regions (302a, b) are formed in the wall (303) of the cylindrical body with the winding.

3. The method according to Claim 2, in which a center region (202) of the mat (101) is wetted in part and at least one separating layer (305) is formed in the wall (303) of the cylindrical body with the winding.

4. The method according to one of Claims 1 to 3, in which an end region of the mat (101) is wetted in part and an outer layer (401) is formed in the wall (303) of the cylindrical body with the winding.

5. The method according to Claim 2, in which a film for forming at least one separating layer (305) is wound in with the wall (303) of the cylindrical body.

6. An apparatus for producing a membrane module (301) from a mat (101) made of hollow fibers (102) for a favorable fluidic blood current flow through the membrane module, wherein a plurality of hollow fibers (102) which have approximately the same length are arranged next to one another to form the mat (101), wherein the apparatus has a metering device for casting compound, wherein the metering device is designed so that it may wet the mat (101) in regions, that the apparatus has a tensioning device, wherein this tensioning device is designed so that it can bias the mat (101), and that the apparatus has a winding device, wherein this winding device is designed so that it can wind the biased mat (101), ***characterized in that*** the metering device has a control unit which is designed so that it can determine and specify a contour for a region of the mat (101) to be wetted, and that it can determine and specify a metered quantity for the casting compound for the region with the determined contour to be wetted, wherein for each of the regions of the mat (101) to be wetted, a wetting contour is respectively determined, which is defined as a function of the results of a numeric fluid flow analysis which calculates a favorable fluidic blood current flow.

7. A membrane module (301) made from hollow fibers (102), wherein a plurality of hollow fibers (102) which have approximately the same length are arranged next to one another to form the mat (101), wherein the mat (101), after it was wetted with a casting compound in regions and was wound on a cylindrical winding core (304), forms a wall (303) of the membrane module (301) in regions with at least one molded region, ***characterized in that*** the contours of the molded regions, which are visible in a longitudinal section through the wall (303), have a curve in regions, wherein the wall (303) has two molded end regions (302a, b) and at least one separating layer (305), wherein additionally the separating layer (305) is formed by a casting compound, wherein the membrane module is produced according to a method according to Claim 1.

8. The membrane module (301) according to claim 7, in which the wall (303) has an outer layer (401) which is formed by a casting compound.

## Revendications

1. Procédé, destiné à la production d'un module membranaire (301) à partir d'une natte (101) en fibres de bois (102) pour assurer un tracé fluidiquement avantageux du flux sanguin à travers le module membranaire, plusieurs fibres de bois (102) d'une longueur approximativement identique placées côte à côte formant la natte (101), lors duquel on humidifie la natte (101) par zones avec une masse de scellement et on enroule la natte (101) humidifiée par zones avec la masse de scellement sur un noyau d'enroulement (304) de forme cylindrique, un corps de forme cylindrique, doté d'une paroi (303) en fibres de bois (102) étant réalisé et la paroi (303) en fibres de bois (102) comportant au moins une zone scellée, pour chacun des zones de la natte (101) qui doivent être humidifiées étant prédéfini respectivement un contour d'humidification, ***caractérisé en ce qu'***on définit le contour d'humidification en fonction des résultats d'un calcul numérique de l'écoulement, qui calcule un trajet fluidiquement avantageux du flux sanguin.

2. Procédé selon la revendication 1, lors duquel on humidifie deux zones marginales (201a, b) opposées de la natte (101) avec la masse de scellement et par l'enroulement, on crée deux zones marginales (302a, b) scellées dans la paroi (303) du corps de forme cylindrique.

3. Procédé selon la revendication 2, lors duquel on humidifie partiellement une zone centrale (202) de la natte (101) et par l'enroulement, on créé au moins une couche de séparation (305) dans la paroi (303) du corps de forme cylindrique.

4. Procédé selon l'une quelconque des revendications 1 à 3, lors duquel on humidifie partiellement une zone d'extrémité de la natte (101) et par l'enroulement, on créé une couche extérieure (401) dans la paroi (303) du corps de forme cylindrique.

5. Procédé selon la revendication 2, lors duquel on enroule un film destiné à la création d'au moins une couche de séparation (305) dans la paroi (303) du corps de forme cylindrique.

6. Dispositif, destiné à la production d'un module membranaire (301) à partir d'une natte (101) en fibres de bois (102) pour assurer un tracé fluidiquement avantageux du flux sanguin à travers le module membranaire, plusieurs fibres de bois (102) d'une longueur approximativement identique placées côte à côte formant la natte (101), le dispositif comportant un système de dosage pour la masse de scellement, ledit système de dosage étant conçu de sorte à pouvoir humidifier la natte (101) par zones, le dispositif comportant un système tendeur, ledit système tendeur étant conçu de sorte à pouvoir tendre préalablement la natte (101) et le dispositif comportant un système d'enroulement, ledit système d'enroulement étant conçu de sorte à pouvoir enrouler la natte (101) préalablement tendue, ***caractérisé en ce que*** le système de dosage comporte un système de commande qui est conçu de sorte à pouvoir déterminer et prédéfinir un contour pour une zone qui doit être humidifiée de la natte (101) et **en ce que** pour la zone qui doit être humidifiée, en plus du contour déterminé, il peut déterminer et prédéfinir une quantité de dosage pour la masse de scellement, pour chacune des zones qui doivent être humidifiées de la natte (101) étant prédéfini chaque fois un contour d'humidification qui est défini en fonction de résultats d'un calcul numérique de l'écoulement qui calcule un trajet fluidiquement avantageux du flux sanguin.

7. Module membranaire (301) en fibres de bois (102), plusieurs fibres de bois (102) d'une longueur approximativement identique placées côte à côte formant la natte (101), après avoir été humidifiée par zones avec une masse de scellement et enroulée sur un noyau d'enroulement (304) de forme cylindrique, la natte (101) formant par zones une paroi (303) du module membranaire (301) avec au moins une zone scellée, ***caractérisé en ce que*** les contours des zones scellées qui sont visibles dans une coupe longitudinale à travers la paroi (303) comportent par zones une courbure, la paroi (303) comportant deux zones marginales (302a, b) scellées, ainsi qu'au moins une couche de séparation (305), par ailleurs la couche de séparation (305) étant formée par une masse de scellement, le module membranaire ayant été produit d'après un procédé selon la revendication 1.

8. Module membranaire (301) selon la revendication 7, sur lequel la paroi (303) comporte une couche extérieure (401) qui est formée par une masse de scellement.
